# EUROPEAN PATENT APPLICATION

(11) **EP 1 693 010 A1**
(43) Date of publication of application: **23.08.2006**
(21) Application number: 05250997.3
(22) Date of filing: 22.02.2005
(51) Int. Cl.: A61B 17/15

(54) **A surgical instrument combination for accurate positioning of a cutting plane**

(71) Applicant: DePuy Orthopädie GmbH, 85551 Helmstetten (DE)
(72) Inventor: Baldewein, Jury, 81673 Munich (DE)
(74) Representative: Belcher, Simon James

(57) **Abstract**

An instrument combination for use in preparing a bone for receiving a component of an orthopaedic joint prosthesis comprises a primary instrument which can be fixed to the bone, and whose position relative to the bone must be determined, the primary instrument having a slot in it defined by a slot reference surface and a slot opposite surface. A plane finder instrument includes a plate having a plate reference surface and a plate surface, with a resiliently deformable element on the plate opposite surface. The plate can be fitted into the slot with the plate reference surface facing towards the slot reference surface, and be urged towards it by the action of the resiliently deformable element against the slot opposite surface.

## Description

This invention relates to an instrument combination for use in preparing a bone for receiving a component of an orthopaedic joint prosthesis.

Preparation of a bone to receive a component of a joint prosthesis involves cutting the bone so that it is configured appropriately to enable the joint prosthesis component to fit to it. The bone can be cut using tools such as saws and drills.

It is important that the cutting steps are performed accurately so that the prosthesis component can be fitted securely to the bone, and so that it is appropriately located to enable the joint to articulate in a way which resembles as closely as possible the articulation of a natural joint.

Instruments for use in preparing a bone to receive a component of a joint prosthesis include guides which can be fitted to the bone, and which define the plane for a cut using a saw, and the axes for forming a bore using a drill. The position of a guide should be determined before a cutting (including drilling) step is performed to ensure that the cutting step is performed accurately.

It is known to determine the position of an instrument during surgery using a tracking detector which can receive position data from a sensor on the instrument. For example, an instrument can have a plurality of emitters or reflectors mounted on it, and the tracking detector can detect radiation from the emitters or reflectors. Information about the relative location of the emitters or reflectors to one another and to the instrument can allow the position of the instrument to be determined from signal that is monitored by the tracking detector.

This invention provides an instrument combination which comprises a primary instrument (for example a guide for a subsequent cutting step) which can be fixed to the bone and a plane finder instrument having a plate which can fit into a slot in the primary instrument, with a resiliently deformable element to urge a reference surface on the plate towards a reference surface in the slot.

Accordingly, in one aspect, the invention provides an instrument combination for use in preparing a bone for receiving a component of an orthopaedic joint prosthesis, which comprises:
a. a primary instrument which can be fixed to the bone, and whose position relative to the bone must be determined, the primary instrument having a slot in it defined by a slot reference surface and a slot opposite surface, and
b. a plane finder instrument which includes a plate having a plate reference surface and a plate surface, with a resiliently deformable element on the plate opposite surface, in which the plate can be fitted into the slot with the plate reference surface facing towards the slot reference surface, and being urged towards it by the action of the resiliently deformable element against the slot opposite surface.

The instrument combination of the invention has the advantage that the plane finder instrument can be fitted securely into slots in primary instruments with a range of slot dimensions (measured between the slot reference and opposite surfaces). Information about the plane of the slot reference surface (in particular, its arrangement within the primary instrument) can be used with information that is obtained from the plane finder instrument about its location to identify the position of the primary instrument. Accordingly, it will generally be preferred for the plane finder instrument to include a sensor which can provide position data to enable the location and orientation of the plane finder instrument to be monitored.

The resiliently deformable element should be capable of elastic deformation when the plate is inserted into the slot, as a result of action against the slot opposite surface as the plate reference surface slides against the slot reference surface. It is preferable that the deformation of the deformable element on insertion of the plate into the slot should be recovered completely when the plate is withdrawn from the slot so that the plane finder instrument can be reused. Accordingly, the deformation of the deformable element which takes place when it is inserted into the slot should not exceed the elastic limit of the material of the element.

The resiliently deformable element can be provided by a part which is separable from the plate, and which is biassed resiliently relative to the plate. For example, the element can comprise a sub-plate which can articulate relative to the plate, for example on guide posts which extend from the plate.

It is particularly preferred however that the resiliently deformable element comprises a tongue which is provided by the material of the plate, bent out of the plane of the plate. The resilient deformation of the tongue can then involve bending at or towards the root of the tongue (where the tongue is connected to the body of the plate). The thickness of the plate at the root of the tongue can be reduced locally in order to facilitate the deformation.

Preferably, the width of the tongue at its root is less than at the point where it contacts the opposite surface of the slot. This can provide greater stability for the connection between the primary instrument and the plane finder instrument. For example, it can be preferred for the ratio of the width of the tongue at its root to the width at the point where it contacts the opposite surface of the slot to be not more than about 0.8, more preferably not more than about 0.7, especially not more than about 0.6.

Preferably, the slot dimension measured between the slot reference surface and the slot opposite surface is at least about 1 mm, more preferably at least about 2 mm. Preferably, the slot dimension measured between the slot reference surface and the slot opposite surface is not more than about 6 mm, more preferably not more than about 5 mm, especially not more than about 4 mm.

Preferably, the thickness of the plate measured between the plate reference surface and the plate opposite surface is at least about 0.5 mm, more preferably, at least about 1.0 mm, especially at least about 1.5 mm. Preferably, the thickness of the plate measured between the plate reference surface and the plate opposite surface is not more than about 3 mm.

Preferably, the difference between (a) the thickness of the plate measured between the plate reference surface and the plate opposite surface, and (b) the slot dimension measured between the slot reference surface and the slot opposite surface, is at least about 0.5 mm, more preferably at least about 1.0 mm, especially at least about 1.5 mm.

The plate can be chamfered at the edge which is offered to the slot to facilitate entry of the plate into the slot.

Preferably, the areas of the plate and slot reference surfaces which are in contact is at least about 3 cm², more preferably at least about 4 cm².

Preferably, the instrument combination includes a stop to limit the depth of insertion of the plate into the slot. The stop can be provided on the primary instrument, especially in the slot therein. The stop can be provided in the plane finder instrument, especially on the plate.

A sensor in the plane finder instrument for providing position data to enable the location and orientation of the plane finder instrument to be monitored can function optically. For example, a sensor can comprise a plurality of emitters or reflectors mounted on the plane finder instrument, which operates with a tracking detector which can detect radiation from the emitters or reflectors. Information about the relative location of the emitters or reflectors to one another and to the instrument can allow the position of the instrument to be determined from signal that is monitored by the tracking detector.

It can be particularly preferred for the sensor to respond to an electromagnetic field to generate position data relating to the instrument. The sensor can comprise a Hall effect device, coils, or other antennae. An example of a suitable coils sensor is disclosed in US-A-2003/0120150. The disclosed sensor includes at least one sensing coil which can generate a signal when it moves within an electromagnetic field transmitted by a local transmitter. The disclosed coil sensor is able to provide position information in multiple degrees of freedom, including up to six degrees of freedom. It can be preferred for the plane finder instrument to provide position information in six degrees of freedom although less detailed information can be appropriate for some applications, for example at least three degrees of freedom, preferably at least four degrees of freedom, more preferably at least five degrees of freedom.

Preferably, the instrument combination of the invention is used as part of a tracking system which uses a local magnetic field generator, provided on a pad which is adapted to be affixed to a surface of the body of the patient. The pad can include a plurality of concentric orthogonal magnetic field generating coils. A driving antenna can be provided to radiate a radio frequency (RF) electromagnetic field. The pad can include a power coil which is coupled to receive the RF electromagnetic field and thereby to provide power for generating the magnetic field. Alternatively, the pad can include an internal power source to provide power for generating the magnetic field. It can however be particularly preferred for the pad to be connected by means of conductors to a source of electrical power.

A local magnetic field generator which is provided on a pad can be used in conjunction with a position sensor which is implanted in a patient's body, to provide information about the patient, in particular as to the location and orientation of the part of the body in which the sensor is implanted.

Tracking systems which comprise a pad on which a magnetic field can be generated and at least one position transducer are disclosed in UK and US patent applications which are filed with the present application, entitled Reference Pad for Position Sensing. Subject matter that is disclosed in the specifications of the patents and patent applications referred to in this paragraph is incorporated in this specification for all purposes by these references.

The plane finder instrument can include an ADC (Analogue-to-Digital Convertor) for digitising the output of the sensor. The instrument may also be hard wired for output to a computer system. In that case either an analogue or a digital signal may be transmitted over the wires. If an analogue signal is used, no ADC is required.

Preferably, the plane finder instrument includes a processor for processing the output of the ADC using predetermined calibration data of the position of the plate relative to the sensor to calculate the position of the plate.

Calibration can be carried out at the point of manufacture. This allows the instrument to be used with any system without requiring local calibration. The processor may be implemented as a discrete component or integrated with the ADC.

The processor may be powered in a number of ways. For example, it may be powered by inductive coupling with an external electromagnetic field, by batteries or through a wired link if one is present.

Preferably, the processor is adapted to monitor the location of the sensor relative to the plate. The plane finder instrument may further comprise a switch connected to an input of the processor. Operation of the switch by a user can then cause the instrument to output the current position data. This allows the position to be stored and analysed at the command of the user when the instrument in the correct position.

Preferably, the plane finder instrument further comprises a power source and a transmitter for transmitting the position data wirelessly. Preferably, the transmitter is contained within the handle of the plane finder instrument. The power source may be a battery contained within the handle. The instrument may also comprise an output socket for outputting position data over a wired connection.

Accordingly, it can be preferred that the plane finder instrument includes a handle. The handle can contain components such as a sensor, an analogue to digital converter, a data processor and a power source (especially a battery). Any of the above components can be incorporated in the handle. Preferably, all of the above components are incorporated in the handle.

The handle can be made from polymeric material. Preferably, components which are contained in the handle are located in a chamber within the handle which is sealed against ingress of liquid and other contaminants. The chamber can have external contacts for mounting an external battery pack. Suitable materials for the handle include polymers, especially polyolefins, polyamides and polyetheretherketones, optionally with fibre reinforcement. The materials should be capable of withstanding conditions to which the instrument will be exposed during use and during cleaning operations, for example involving exposure to radiation or to ultrasonic vibration (for example 20 to 200 kHz for 15 minutes) or to elevated temperatures and pressures. The materials should be sufficiently rigid that there is minimal relative movement between the plate and a sensor located within the handle.

The plate of the plane finder instrument should be made from a material which is sufficiently rigid for there to be minimal bending of the plate (other than of the deformable element). Metals can be suitable, provided that the material does not interfere with any electromagnetic field or resulting signal. When the deformable element is provided by a tongue which is connected to the body of the plate, some flexibility of the material of the plate will be required. Polymeric materials might also be used.

Magnetic fields in the vicinity of an object being tracked can be distored by the presence of metal or other magnetically-responsive articles. For example, in a typical surgical environment, there can be a substantial amount of conductive and permeable material, including basic and ancillary equipment (operating tables, carts, movable lamps, etc.), as well as invasive surgery apparatus (scalpels, scissors, etc). The magnetic fields produced by the field generator coils may generate eddy currents in such articles, and the eddy currents then cause a parasitic magnetic field to be radiated. Such parasitic fields and other types of distortion can lead to errors in determining the position of the object being tracked.

In order to alleviate this problem, the elements of a tracking system and other articles used in the vicinity of the tracking system are typically made of non-metallic materials when possible, or of metallic materials with low permeability and conductivity. In addition, a system controlling computer might be programmed to detect and compensate for the effects of metal objects in the vicinity of the surgical site. Suitable methods for such detection and compensation are disclosed in US-6147480, US-6373240, US-A-2004/0240240 and US-A-2005/0024043. Subject matter that is disclosed in the specification of those patents and patent applications is incorporated in this specification for all purposes by these references.

The plate should be fastened to the handle with sufficient rigidity to ensure that there is minimal relative movement between the two. The plate might be formed as one piece with the handle. The plate can be fastened permanently to the handle, for example by bonding, especially by means of an adhesive, or by a technique such as welding. The plate can be fastened to the handle by means of one or more fasteners such as a threaded screw or a rivet. The use of fastener that is removable can have the advantage that the plate can be detached from the handle, for example for replacement. This can be useful because the plate can be susceptible to damage, for example as a result of being bent. This can result in the calibration of the plane finder tool being lost. Provided that replacement plates have reproducible dimensions, assembly of the plate with the handle can reestablish the calibration of the instrument.

The primary instrument can be an instrument is a guide for a cutting tool. In particular, the slot in the primary instrument can be a slot for guiding a saw in a cutting step of preparing a bone in a surgical procedure. The slot will be dimensioned so that the saw blade is a sliding fit in the slot, as is well known. The cutting tool can have one or more bores in it for guiding a drill when forming a bore in a bone. The primary instrument can be fastened directly to the bone, for example by means of fasteners which extend through holes in the instrument. The use of such fasteners, such as screws and pins, to fasten instruments to bone is well established. The cutting tool can be fastened to the bone indirectly, through mounting on a rod which can be intramedullary rod or an extramedullary rod.

The material from which the primary instrument is made will be selected according to the intended function of that instrument. When the instrument is intended for use as a guide for a cutting tool, it should be made from a material that is sufficiently hard to ensure that the instrument is not damaged during the cutting step. Suitable materials for such instruments are well known, including certain stainless steels.

Embodiments of the present invention will now be described by way of example with reference to the accompanying drawings, in which:
Figure 1 is an isometric view of a tibial cutting block and a plane finder instrument.
Figures 2a and 2b are side views, partially in section, through the cutting block and plane finder instrument shown in Figure 1.
Figure 3 is a sectional elevation through the plane finder instrument shown in Figure 1, before the resilient tongue is bent out of the plane of the plate.
Figure 4 is a plan view of the plate of the plane finder instrument shown in Figure 3.

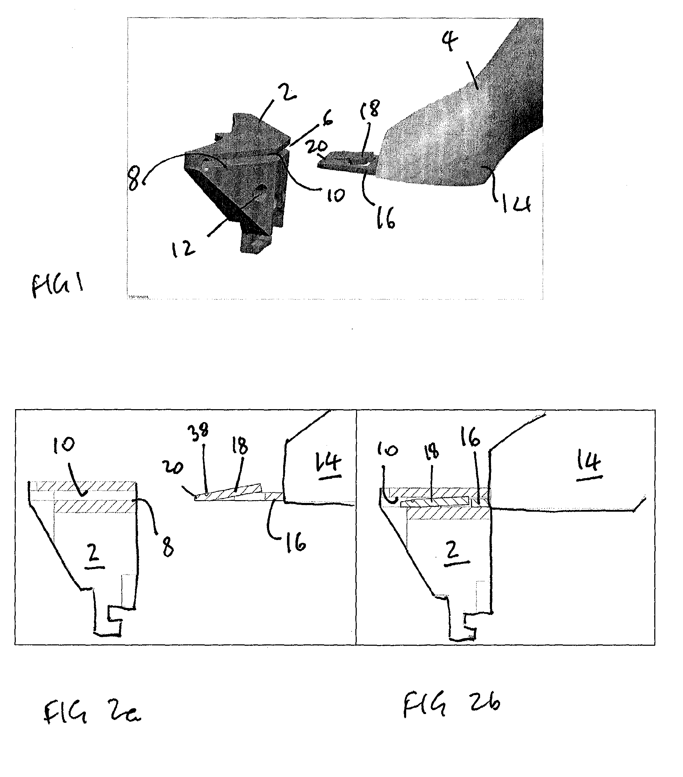

Referring to the drawings, Figure 1 shows a tibial cutting block 2 and a plane finder instrument 4. The tibial cutting block is a primary instrument which can be used during a knee replacement procedure to guide the blade of a saw which is used to cut the tibia so that it can receive the tibial component of a knee joint prosthesis. The tibial cutting block has a slot 6 formed in it defined by a lower reference surface 8 and an upper opposite surface 10. The slot 6 can receive the blade of a saw which is guided by the reference surface 8 of the slot during the bone cutting step.

The tibial cutting block has bores through which fixation pins can be inserted. The fixation pins can be used to fasten the cutting block to the tibia.

In order to ensure accurate reception of the tibia, it is important to ensure that the cutting block is correctly located and fixed to the tibia prior to performing the cutting step. The plane finder instrument 4 is used to check the proper location. The plane finder can be used before the cutting block is finally fixed to the tibia, for example when just one fixation pin has been deployed. Alternatively or in addition, the plane finder can be used after the cutting block has been fixed to the tibia against any additional movement by way of verification of correct positioning.

The plane finder instrument has a handle 14 and a plate 16. The plate has a tongue 18 which is bent out of the plane of the plate. The plate is chamfered at its leading edge 20.

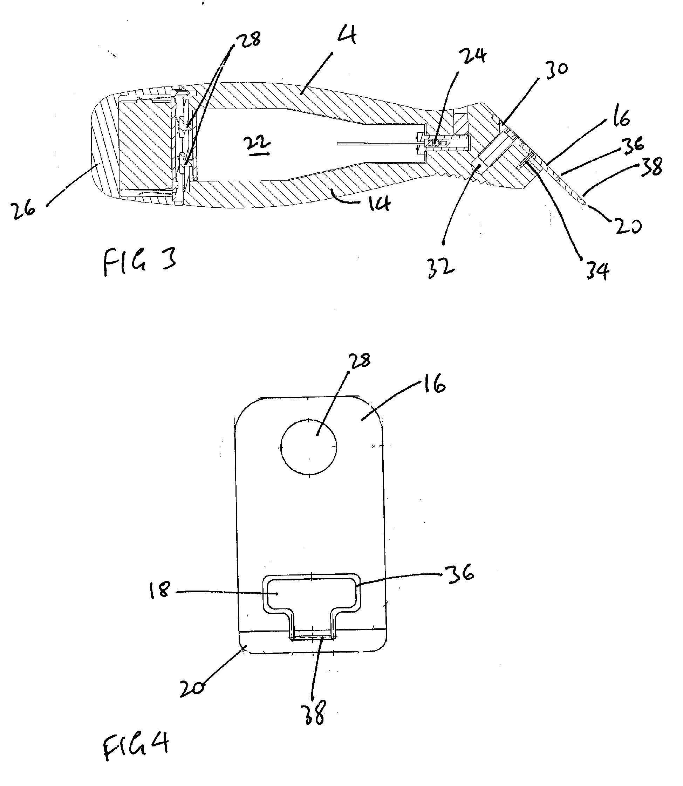

Referring now to Figure 3, the handle 14 of the plane finder instrument 4 consists of a moulded body formed from carbon fibre reinforced polyetheretherketone. The handle is hollow, with the space 22 within the handle containing sensor and transistor components.

In particular, the handle contains a coil sensor of the type disclosed in US-A-2003/0120150. This sensor includes sensing coils which can generate a signal when the handle is moved within an electromagnetic field. The coil sensor is able to provide position information in multiple degrees of freedom. The sensor 24 is fixed rigidly within the handle so that it is incapable of movement within the handle during normal operation.

The handle also contains an analogue 2 digital converter for the signal generated by the sensor 24, and a processor. The processor may be a general purpose processor, an application specific integrated circuit (ASIC), a programmable digital signal processor (DSP), or any suitable alternative. The analogue signal from the position sensor is converted to a digital value by the ADC and supplied to the processor which can then calculate the position and orientation of the plane finder instrument.

The plane finder instrument 4 also includes a battery pack 26 which can be mounted detachably on the handle. The main body portion of the handle has contacts 28 through which power from the battery can be supplied to the components within the handle.

Preferably, the handle includes a transmitter by which the signal originating from the sensor 24 can be transmitted to a system control unit. Such position monitoring systems are known, for example as disclosed in US-A-2003/0120150, US-6332089 and US-6239724.

The plate 16 has a round opening 28 formed in it which can receive the countersunk head of a fastening bolt 30. The handle 14 has a threaded bore 32 formed in it in which the threaded shank of the fastener 30 can be received.

An alignment pin 34 is provided in the plate engaging surface of the handle 14, press fitted into a small bore opening therein. The protruding end of the pin 34 is received in a blind bore in the plate 16.

The angle between the plane of the plate 16 and the axis of the handle 14 is between 30 and 60 degrees, preferably about 45 degrees.

Referring now to Figure 4 which shows the plate 16. The tongue 18 is formed by cutting a slot 36. The tongue is connected to the plate at its root 38, where a groove is formed in the plate to define a line of weakness. The line of weakness provided by the groove 38 facilitates bending of the tongue.

In use, the tongue 18 is offered to the slot 6 as shown in Figure 2a. The tongue 18 is bent out of the plane of the plate 16. The chamfered edge 20 of the plate facilitates insertion of the plate into the slot.

The plate is pushed into the slot until the adjacent end face of the handle 14 abuts the face of the cutting block 2. This limits the depth of insertion of the plate into the slot.

The depth of the slot is slightly greater than the thickness of the material of the plate. However, the plate is only able to fit into the slot if the tongue 18 is bent downwardly, towards the position in which it is in the plane of the plate. It is bent in this fashion as a result of contact with the upper surface 10 of the slot 6, as shown in Figure 2b. The action of the tongue 18 against the upper surface 10 of the slot 6 causes the lower surface of the plate to press against the lower referenced surface 8 of the slot. In this way, the plane finder instrument 4 is able to provide information as to the location and orientation of the lower referenced surface 8 of the slot.

Information concerning the location and orientation of the plate, and therefore of the lower referenced surface 8 of the slot 6 in the cutting block 2, can be derived from a signal from the sensor 24 in the handle 14. This information can be transmitted to a system control unit. It can be compared with surgical plan information which is used by the surgeon when planning and performing the surgical procedure.

## Claims

1. An instrument combination for use in preparing a bone for receiving a component of an orthopaedic joint prosthesis, which comprises:
a. a primary instrument which can be fixed to the bone, and whose position relative to the bone must be determined, the primary instrument having a slot in it defined by a slot reference surface and a slot opposite surface, and
b. a plane finder instrument which includes a plate having a plate reference surface and a plate surface, with a resiliently deformable element on the plate opposite surface, in which the plate can be fitted into the slot with the plate reference surface facing towards the slot reference surface, and being urged towards it by the action of the resiliently deformable element against the slot opposite surface.

2. An instrument combination as claimed in claim 1, in which the plane finder instrument includes a sensor which can provide position data to enable the location and orientation of the plane finder instrument to be monitored.

3. An instrument combination as claimed in claim 2, in which the sensor can respond to an electromagnetic field, to generate position data relating to the instrument.

4. An instrument combination as claimed in claim 3, in which the plane finder instrument includes an analogue to digital converter for digitising the data that is generated by the sensor.

5. An instrument combination as claimed in claim 3, in which the plane finder instrument includes a transmitter for transmitting the position data wirelessly.

6. An instrument combination as claimed in claim 5, in which the plane finder instrument includes a handle, and in which the transmitter is contained in the handle.

7. An instrument combination as claimed in claim 3, which includes a device for generating an electromagnetic field.

8. An instrument combination as claimed in claim 7, in which the electromagnetic field generating device generates an amplitude of not more than about * at a range of 200 mm.

9. An instrument combination as claimed in claim 1, in which includes a stop to limit the depth of insertion of the plate into the slot.

10. An instrument combination as claimed in claim 1, in which the resiliently deformable element comprises a tongue which is provided by the material of the plate, bent out of the plane of the plate.

11. An instrument combination as claimed in claim 10, in which the width of the tongue at its root where it is connected to the body of the plate is less than at the point where it contacts the opposite surface of the slot.

12. An instrument combination as claimed in claim 1, in which the slot dimension measured between the slot reference surface and the slot opposite surface is at least about 1 mm.

13. An instrument combination as claimed in claim 1, in which the slot dimension measured between the slot reference surface and the slot opposite surface is not more than about 5 mm.

14. An instrument combination as claimed in claim 1, in which the thickness of the plate measured between the plate reference surface and the plate opposite surface is at least about 1 mm.

15. An instrument combination as claimed in claim 1, in which the thickness of the plate measured between the plate reference surface and the plate opposite surface is not more than about 3 mm.

16. An instrument combination as claimed in claim 1, in which the difference between (a) the thickness of the plate measured between the plate reference surface and the plate opposite surface, and (b) the slot dimension measured between the slot reference surface and the slot opposite surface, is at least about 1 mm.

17. An instrument combination as claimed in claim 1, in which the primary instrument is a guide for a cutting tool.

18. An instrument combination as claimed in claim 1, in which the areas of the plate and slot reference surfaces which are in contact is at least about 3 cm².
